# EUROPEAN PATENT APPLICATION

(11) **EP 4 133 995 A1**
(43) Date of publication of application: **15.02.2023**
(21) Application number: 22189281.3
(22) Date of filing: 08.08.2022
(51) Int. Cl.: A61B 5/00, A61B 5/291, A61B 5/256

(54) **ELECTRODE SUPPORT DEVICE FOR RECORDING ELECTROENCEPHALOGRAPHIC SIGNALS**

(30) Priority: 11.08.2021 IT 202100021785; 07.09.2021 IT 202100023132
(71) Applicant: Spes Medica SRL, 16153 Genova (IT)
(72) Inventor: Mistretta, Matteo, 16153 Genova (GE) (IT); Zaytsev, Mykyta, 16153 Genova (GE) (IT)
(74) Representative: Bottino, Giovanni

(57) **Abstract**

Electrode support device for recording electroencephalographic signals, comprising a cap element (1) configured to at least partially surround the skull of a patient, so that said cap element (1) has an inner surface in contact with the patient's scalp, which cap element (1) is configured so as to place in pre-defined zones a plurality of electrodes in contact with the patient's scalp.

Said cap element (1) consists of flexible polymeric material.

## Description

The present invention relates to an electrode support device for recording electroencephalographic signals, comprising a cap element configured to at least partially surround the skull of a patient.

The presence of the cap element allows to create a wearable device, which, in the worn condition, has the cap element with an inner surface in contact with the patient's scalp.

Furthermore, the cap element is configured so as to place, in pre-defined zones, a plurality of electrodes in contact with the patient's scalp. The one just described is the common configuration of the electrode support devices known in the state of the art, used for the detection of the electroencephalography traces (EEG), that is, which provide for the application of the electrodes in contact with the patient's skin.

Such devices generally comprise cap elements
made of fabric, which have openings at the terminals of the electrodes that must be in contact with the scalp, so as to flexibly support the electrodes.

The electrodes are positioned on the cap element, so that, in the worn condition of the device, the electrodes are positioned in strategic positions.

Currently the positioning of the electrodes is calculated with the known system 10-20, which envisages fixed points of positioning of the electrodes, calculated on the basis of predefined and predetermined distances with respect to specific reference points.

In particular, the system 10-20 provides for firstly identifying two reference points, one placed above the user's nose and one placed at the nape.

The distance along the patient's cranial surface between these two points is measured and the positioning points along the line joining the two points at pre-established intervals are identified.

In a completely similar way, two reference points at the patient's ears are identified, to then calculate the positioning points.

The system 10-20 provides for identifying then further positioning points at the two hemispheres of the brain and at the line that identifies the cranial circumference of the patient.

The support devices known in the state of the art therefore allow the electrodes to be positioned in the correct position, simply by wearing the cap element.

For the function of flexibility and wearability to be performed, the cap elements belonging to the devices known in the state of the art, are made of fabric, so as to be comfortable on the patient's skull and, at the same time, have a certain elasticity, so as to deform, adapt to the patient's skull and keep the electrodes adherent to the scalp.

The adhesion of the electrodes is obviously a key factor, as it allows performing a correct detection of the EEG signals.

For this reason, the cap elements are made of fabric, generally cotton, as it is the only material that fulfils all the required functionalities, and whose elasticity can be modified based on the texture of the fabric and the fibres used.

The use of fabric, however, does not allow to obtain disposable caps, as the production is too complex and expensive in order to be able to make "disposable" cap elements.

This aspect is particularly disadvantageous in that it requires using the same cap for different patients, which must be cleaned and disinfected after each use.

The cleaning and disinfection procedure is particularly time-consuming and must be a validated process, as at each cleaning and disinfection all the electrodes individually must be cleaned, disinfected and rinsed and finally it must be waited for them to dry; this also implies having many pieces available to be able to continue with the tests.

There is therefore a need, not satisfied by the systems known to the state of the art, to manufacture an electrode support device that is disposable and that has low production costs, maintaining wearability and functionality characteristics of the cap elements known to the state of the art unchanged and that allows to avoid any possibility of cross contamination of the patients and of the healthcare professionals.

The present invention achieves the above purposes by manufacturing a device as described above, wherein the cap element consists of flexible polymeric material.

The use of flexible polymeric material makes it possible to obtain a cap element that is easy to manufacture, with low production costs and which can therefore be used as a disposable cap.

In addition, a flexible, soft and elastic cap is obtained, which has the prerogative, precisely because of the nature of the material used, not to have sharp edges in contact with the skin of the patient who wears it, thus obtaining a high comfort in the worn condition of the cap.

According to a possible embodiment, the polymeric material consists of transparent material.

The use of transparent material represents a particularly innovative aspect compared to the reusable and disposable caps known in the state of the art, which are made of opaque coloured materials.

The presence of transparent material allows first of all to check the correct positioning of each electrode, as well as the correct contact of each electrode with the patient's scalp.

In addition, the transparent material allows, again in the worn condition of the cap, to check in detail the positioning zone of the cap, the patient's skull, in order to identify areas with trauma, bleeding, as well as to manage the hair in a simple way for a correct positioning of the electrodes.

Preferably the polymeric material consists of polyurethane.

The use of flexible synthetic material certainly has advantages from the point of view of production and of the possibility of manufacturing disposable caps, nevertheless, in order to efficiently replace the fabric used in the caps known in the state of the art, it is necessary to give the polymeric material the right elasticity and flexibility.

In fact, the polymeric material must have sufficient elastic characteristics to deform and adapt to the patient's skull, but at the same time it must be sufficiently rigid to ensure the correct pressure of the electrodes on the patient's skull.

To obtain this result, advantageously the cap element has holes and/or cuts so as to create discontinuity zones of the material constituting the cap element itself.

Thanks to this configuration, it is possible to use a flexible polymeric material sufficiently rigid to ensure the contact of the electrodes, guaranteeing the elasticity of the cap element thanks to the mechanical stretchiness obtained from these discontinuity zones.

The presence of the discontinuity zones also has the advantage of concentrating, in the worn condition of the cap, the deformation of the cap element precisely at these discontinuity zones, so as to avoid creating stresses on the electrodes.

The holes or the cuts can be of any shape known to the state of the art, even of different shapes within the same cap element, as long as they perform the function of creating discontinuities and stretchiness zones of the flexible polymeric material.

Advantageously, the discontinuity zones are homogeneously distributed on the surface of the cap element.

In order to obtain the best compromise between rigidity and elasticity of the flexible polymeric material used, it is essential to balance the correct amount of full zones and empty zones of the cap element, i.e., to carefully evaluate the total dimensions of the holes and/or of the cuts made.

Preferably, the totality of the areas of the discontinuity zones has a value comprised between 5% and 25% of the total surface of the cap element.

In addition to the features just described, it is specified that the electrode support device object of the present invention may envisage two main embodiments, a first embodiment, in which the electrodes are separated from the cap element and applied before wearing the device, and a second embodiment, described below, in which the electrodes are integrated into the cap element.

The two embodiments may be provided alternatively or in combination, i.e., the support device object of the present invention may provide for the electrodes to be separated from the cap element, or the elements integrated in the cap element, as well as part of the separated electrodes and part of the integrated electrodes.

One of the objects of the present invention is in fact to manufacture an electrode support device, in which the electrodes can be integrated or integrable, based on the operational needs.

According to the first embodiment, the cap element has through holes configured for housing at least part of the electrodes.

The holes are used to allow the contact between the contact part of the electrodes and the patient's skull.

The cap element just described can thus be provided in combination with the known state-of-the-art electrodes or, as will be described later, with specific flexible material electrodes printed by using conductive inks, which can be fixed on the cap element in any one of the known state-of-the-art manners, exactly as it happens for the fabric cap elements.

Preferably, the device object of the present invention envisages a specific electrode assembly.

In particular, the electrode assembly consists of a plurality of branches, wherein each branch has a contact part and a transmission part.

Furthermore, each branch has a flexible support element of at least one conductive element, which flexible support element has a head terminal and a tail terminal.

The conductive element has at least one head contact and at least one tail contact, arranged respectively at the head terminal and the tail terminal of the flexible support element and said conductive element consists of a conductive track made of at least one layer of conductive ink deposited on the flexible support element.

The electrode assembly just described therefore has low production costs, the conductive parts are made of conductive ink which is deposited on a flexible polymeric material.

An example of such an electrode is described within document WO2018/122824, the content of which is to be considered as an integral part of this patent application.

It is evident that, thanks to this electrode assembly, a device can be made in which not only the cap element, but also the electrodes are disposable and replaceable for each test and for each patient.

According to a preferred embodiment, means for fixing the contact part of the branches to the edges of the through holes of the cap element are provided.

Both mechanical fixing means, such as adhesive tapes or the like, and chemical-physical fixing means, such as gluing materials or the like, may be envisaged.

The possibility of fixing only the contact part, is a particularly advantageous aspect since the deformation of the cap element, caused while the cap itself is being worn, is not transmitted to the transmission part, so it avoids any deformation of the latter that could cause distortions of the detected signal.

As anticipated, the device object of the present invention envisages a second embodiment, in which the electrodes are integrated within the cap element.

According to such an embodiment, the inner surface or the outer surface of the cap element has a plurality of conductive tracks made of at least one conductive ink layer.

It is apparent that such an embodiment is advantageous from both a production and a functional point of view.

From a production point of view, the steps and the processings to be carried out to manufacture the device are limited since both the cap element and the electrodes are produced simultaneously, saving costs and time.

Time is also saved from a functional point of view, since to carry out a test it will not be necessary to fix the electrodes to the cap element, but it will be sufficient to position the cap element on the patient's skull and adapt it so that the electrodes, in the worn condition, are at the points of the skull where the electroencephalographic signals are recorded, preferably according to the system 10-20, described above.

These and other features and advantages of the present invention will become clearer from the following disclosure of some exemplary embodiments illustrated in the accompanying drawings in which:
figure 1 illustrates a view of a possible embodiment of the electrode support device object of the present invention;
figure 2 illustrates an embodiment example of the electrode assembly belonging to the device object of the present invention;
figures 3a and 3b illustrate two views of a possible embodiment of the electrode support device object of the present invention, respectively according to a side view and a top view.

It is specified that the figures appended to the present patent application illustrate some embodiments of the electrode support device object of the present invention, to better understand the advantages and features described therein.

Such embodiments are therefore to be understood as purely illustrative and not limiting the inventive concept of the present invention, namely that of manufacturing an electrode support device that has the same characteristics of softness, flexibility and fitting of the fabric caps, but which can be used as a disposable cap.

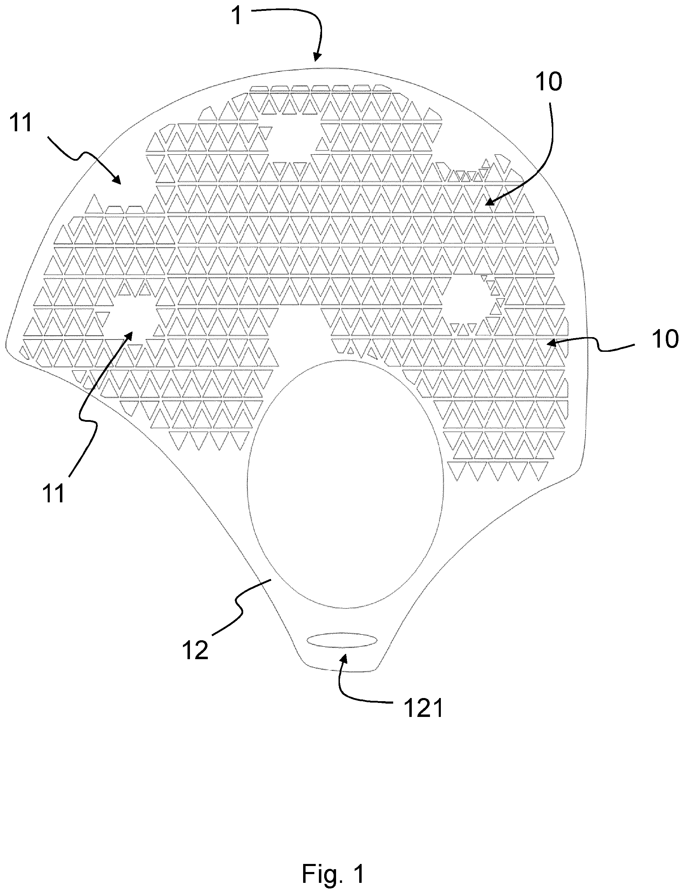

With particular reference to figure 1, a side view of the device object of the present invention is illustrated, comprising a cap element 1 configured to at least partially surround the skull of a patient, so that the cap element 1 has an inner surface in contact with the patient's scalp and an outer surface, shown in figure 1, on which to place a plurality of electrodes.

The cap element 1 consists of flexible polymeric material, in particular of polyurethane.

Furthermore, according to the variant illustrated in figure 1, the cap element 1 has holes 10, made in the thickness of the layer of flexible polymeric material.

The holes 10 allow creating discontinuity zones of the flexible polymeric material.

According to the variant illustrated in the figures, the holes 10 are homogeneously arranged along the surface of the cap element 1.

Furthermore, these holes 10 are triangular in shape, but it is possible to envisage any shape both polygonal and circular, just as it is possible to envisage simple cuts, aimed at forming discontinuity zones of the cap element, that is, to form zones of mechanical stretchiness of the material constituting the cap element 1.

Figure 1 also illustrates the through holes 11 of the cap element 1: these through holes are provided at the positioning points of the electrodes, in the worn condition of the cap.

In the worn condition, in fact, these through holes 10 allow to leave part of the patient's scalp uncovered, in particular the part of the skull in which to position the electrodes, which will then be fixed to the cap element 1.

According to an embodiment not illustrated in the figures, the cap element 1 can only envisage the holes 10 and not the holes 11 as discontinuity zones.

In this case, it is possible to provide that the cap element 1 integrates the electrodes, through the deposition of a layer of conductive ink, aimed at forming a contact part, in contact with the scalp, and a transmission part of the signals detected by the contact part.

Preferably, for construction needs, this conductive ink layer is deposited on the inner surface of the cap element 1.

The conductive ink layer may be deposited directly on the inner surface of the cap element 1, or a coating with special material of the cap element may be envisaged, at least in the zone of deposition of the conductive ink.

Furthermore, it is possible to envisage a further insulation layer at least of the conductive ink transmission part, to prevent this part from being affected by interferences as a result of contact with the scalp.

According to the variant illustrated in figure 1, the cap element 1 further has means for the fixing to a patient's skull, realized by an element 12 aimed at surrounding the patient's ear, which element 12 envisages a slot 121 configured to cooperate with a corresponding tab (not illustrated in the figure) so as to tighten the element 12 under the patient's chin.

According to the variant illustrated in the figures, the cap element 1 is instead envisaged in combination with an electrode assembly.

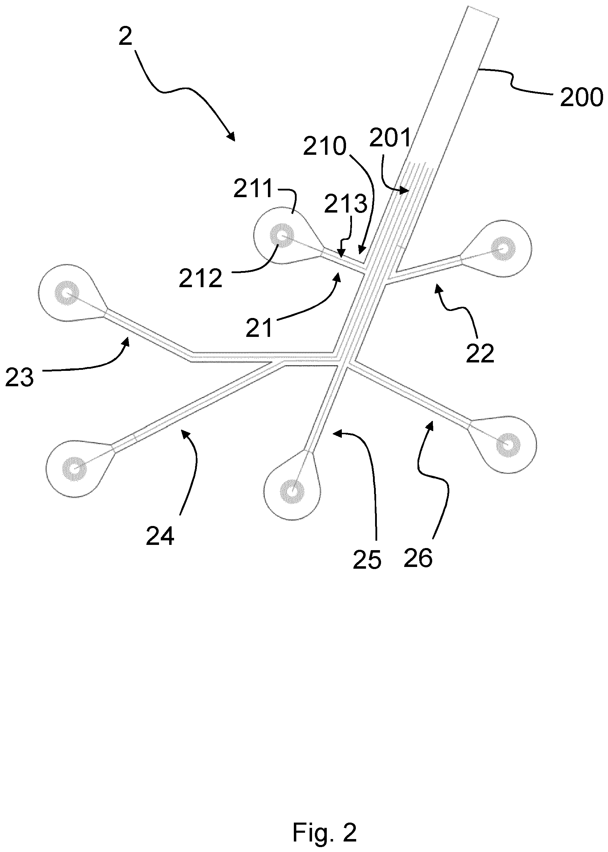

Preferably, said electrode assembly is made according to the variant illustrated in figure 2.

The electrode assembly 2 consists of a plurality of branches 21, 22, 23, 24, 25 and 26.

The electrode assembly 2 has a main branch 200 aimed at transmitting the signals to a processing unit (not illustrated in the figure), signals detected by the branches 21-26 departing from the main branch 200.

The branches 21-26 are made in a similar way, only the dimensions and lengths vary, so as to be able to place the electrodes, in the worn condition of the cap, according to the system 10-20.

For this reason, for the sake of illustrative and expository simplicity, the branch 21 will be described in detail, but it is clear how this description can be considered also for the remaining branches 22-26.

In particular, the branch 21 has a contact part, provided in contact with the scalp of the patient, and a transmission part.

Furthermore, the branch 21 consists of a flexible support element of at least one conductive element, which flexible support element has a head terminal 211 and a tail terminal 210.

Similarly, the conductive element has at least one head contact 212 and at least one tail contact 213, respectively arranged at the head terminal 211 and the tail terminal 210 of the flexible support element.

Furthermore, the conductive element consists of a conductive track made of a layer of conductive ink deposited on the flexible support element.

As illustrated in figure 2, each tail contact 213 of each branch is joined to the conductive tracks 201 provided on the main branch 200, such that the signals detected by the head contacts are transmitted to the tracks 201, to then be processed by the processing unit (not illustrated in the figure).

According to a preferred embodiment, only the head terminal 211 of each branch 21-26 is fixed to the cap element 1, in particular it is possible to fix the edges of this head terminal 211 with the edges of the through holes 11 of the cap element.

It follows that only the head terminal 211 will be integral with the surface of the cap element 1, whereas the tail terminals 210 and the main branch 200 will not be fixed, so they will not undergo the deformations of the cap element 1, in the worn condition.

Furthermore, the head terminals 211 will tend to be pressed towards the scalp of the patient in the worn condition of the device object of the present invention.

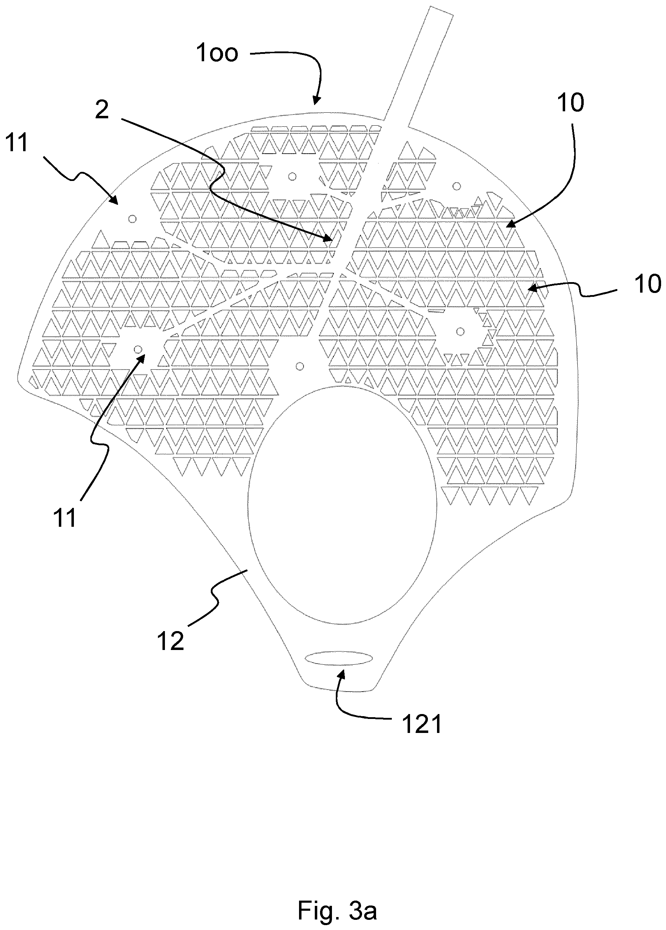

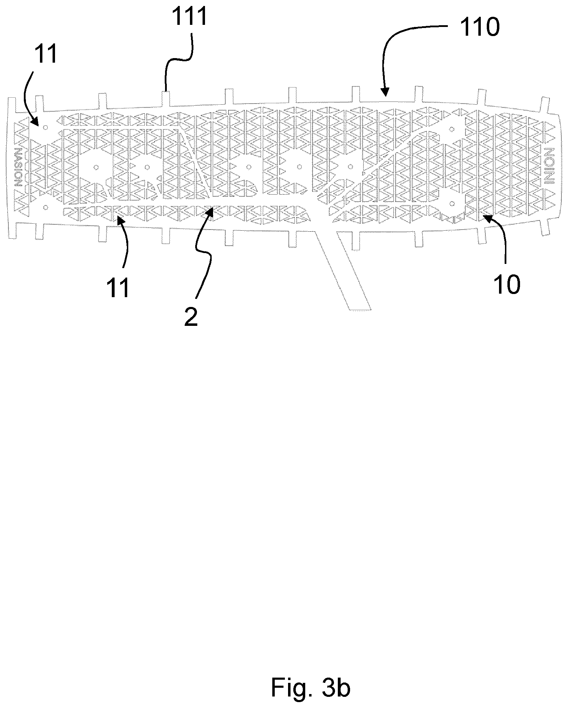

Figures 3a and 3b illustrate two views of the device object of the present invention, wherein the cap element 1 of figure 1 is envisaged in combination with the electrode assembly 2 of figure 2, i.e., wherein the electrode assembly 2 is fixed to the cap element.

Obviously, the conductive tracks of the electrode assembly 2 are not visible in figures 3a and 3b, as they face in the direction of the patient's skull.

Advantageously, the cap element 1 is made up of three parts that are fixed together between them, in a manner completely similar to what happens in the devices known in the state of the art, in which three flaps of fabric are sewn together to give the fabric cap element the round shape.

In particular, with regard to the device object of the present invention, the cap element 1 consists of two side parts 100, as illustrated in figure 3a, respectively one for the left part (that illustrated in figure 3a) and one for the right part of the patient's skull.

The third part consists of an upper band 110, illustrated in figure 3b, which is fixed to the two side parts 100.

The edges of the upper band 110 have a plurality of flaps of material 111, preferably obtained as one piece with the upper band 110, configured to facilitate and maintain the coupling between the upper band 110 and the side parts 100 stable.

While the invention is susceptible to various modifications and alternative constructions, some preferred embodiments have been shown in the drawings and disclosed in detail.

It should be understood, however, that there is no intention to limit the invention to the specific illustrated embodiment but, on the contrary, the aim is to cover all the modifications, alternative constructions and equivalents falling within the scope of the invention as defined in the claims.

The use of "for example", "etc." or "or" refers to non-exclusive non-limiting alternatives, unless otherwise stated.

The use of "includes" means "includes but is not limited to", unless otherwise stated.

## Claims

1. Electrode support device for recording electroencephalographic signals, comprising a cap element (1) configured to at least partially surround the skull of a patient, so that said cap element (1) has an inner surface in contact with the patient's scalp, which cap element (1) is configured so as to place in pre-defined zones a plurality of electrodes in contact with the patient's scalp,
**characterized in that**
said cap element (1) consists of flexible polymeric material.

2. Device according to claim 1, wherein said polymeric material consists of transparent material.

3. Device according to claim 1 or claim 2, wherein said polymeric material consists of polyurethane.

4. Device according to one or more of the preceding claims, wherein the cap element (1) has holes (10) and/or cuts, so as to create discontinuity zones of the material constituting said cap element (1).

5. Device according to claim 4, wherein the discontinuity zones are homogeneously distributed on the surface of said cap element (1).

6. Device according to one or more of the preceding claims, wherein the totality of the areas of said discontinuity zones has a value comprised between 5% and 25% of the total surface of the cap element (1).

7. Device according to one or more of the preceding claims, wherein said cap element (1) has through holes (11) configured for housing at least part of the electrodes.

8. Device according to one or more of the preceding claims, wherein an electrode assembly (2) is provided, which electrode assembly (2) consists of a plurality of branches (21-26), each branch having a contact part and a transmission part,
each branch (21-26) having a flexible support element of at least one conductive element, which flexible support element has a head terminal (211) and a tail terminal (210),
the conductive element having at least one head contact (212) and at least one tail contact (213), respectively arranged at the head terminal (211) and the tail terminal (210) of the flexible support element, said conductive element being constituted by a conductive track made of a layer of conductive ink deposited on the flexible support element.

9. Device according to claim 8, wherein means for fixing said contact part of the branches to the edges of said through holes (11) of the cap element (1) are provided.

10. Device according to one or more of the preceding claims, wherein said inner surface or the outer surface of the cap element has a plurality of conductive tracks made of at least one conductive ink layer.
